(19) Europäisches Patentamt
European Patent Office
Office européen des brevets

(11) **EP 4 025 125 B1**

(12) **EUROPEAN PATENT SPECIFICATION**

(45) Date of publication and mention of the grant of the patent:
**06.08.2025 Bulletin 2025/32**

(21) Application number: **20750816.9**

(22) Date of filing: **09.06.2020**

(51) International Patent Classification (IPC):
*A61B 5/097* (2006.01)    *A61B 5/08* (2006.01)
*A61B 5/00* (2006.01)

(52) Cooperative Patent Classification (CPC):
**A61B 5/097; A61B 5/082; A61B 5/4238**

(86) International application number:
**PCT/US2020/036793**

(87) International publication number:
**WO 2021/021299 (04.02.2021 Gazette 2021/05)**

(54) **BREATH ANALYZER**

ATEMANALYSATOR

ANALYSEUR D'HALEINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.07.2019   US 201962879345 P**
**10.12.2019   PCT/US2019/065550**

(43) Date of publication of application:
**13.07.2022   Bulletin 2022/28**

(73) Proprietors:
• **Heteron Biotechnologies, LLC**
**Setauket, New York 11733 (US)**
• **Rigas, Anastasia**
**Setauket, New York 11733 (US)**

(72) Inventors:
• **CHIANG, Hao-Chun**
**New York 11791 (US)**
• **LEVON, Kalle**
**Brooklyn, New York 11238 (US)**
• **ARTAN, Nabi Sertac**
**Brooklyn, New York 11223 (US)**
• **AMOAKO, Edward**
**New York 11368 (US)**
• **RIGAS, Anastasia**
**New York 11733 (US)**

(74) Representative: **Herrero & Asociados, S.L.**
**Edificio Aqua - Agustín de Foxá, 4-10**
**28036 Madrid (ES)**

(56) References cited:
**WO-A2-2012/125745     CN-A- 109 164 140**
**JP-A- 2012 202 874**

**Description**

**RELATED APPLICATIONS**

This application claims priority to International PCT Application No. PCT/US2019/065550, filed December 10, 2019, and also claims priority to U.S. Provisional Application No. 62/879,345, filed July 26, 2019.

**FIELD OF THE INVENTION**

The present application relates generally to a breath analyzer and breath test method for detecting gases in a human breath sample to determine the presence or absence of disease in a subject's digestive tract. In some instances, the disease can include, but is not limited to, *H. Pylori.*

**BACKGROUND OF THE INVENTION**

*Helicobacter Pylori* (*"H. Pylori"*), which affects about two-thirds of the world population, causes chronic gastritis and the majority of peptic ulcers, and is the major cause of gastric cancer, the third leading cause of cancer deaths worldwide. Although the prevalence of *H. Pylori* has been decreasing in western countries since its discovery in 1982, it has remained at very high levels in Eastern Europe, Asia and Asia-Pacific, affecting more than 80% of young adults and children. Eradication of *H. Pylori* and prevention of gastric cancer in the majority of cases is achieved with a combination of antibiotics and proton pump inhibitors (PPIs). One important property of *H. Pylori* is its abundance of the enzyme urease, which hydrolyzes urea. When *H. Pylori* in the stomach is exposed to urea, it converts urea to $CO_2$ and $NH_3$, both of which reach the lungs through the bloodstream and are exhaled in the breath. The breakdown of urea is described by the following equation:

$$CO(NH_2)_2 + H_2O \xrightarrow{\textit{-urease}} CO_2 + 2NH_3$$

Detection of *H. Pylori* can be achieved through invasive and non-invasive diagnostic methods. The invasive methods include 1) upper gastrointestinal endoscopy with biopsies for culture and rapid urease test (CLO test); and 2) a blood test for serum antibodies to *H. Pylori.* The advantages of invasive methods are the accuracy of diagnosis with the examination of the tissue from the stomach which contains *H. Pylori,* and the accuracy of the serum antibody. The disadvantages are a) the high cost, inconvenience, and risk of the endoscopy; b) misdiagnosis due to failure to obtain tissue from the actual site of the stomach which contains *H. Pylori*; and c) the inability of serum antibodies to *H. Pylori* to confirm eradication (they persist for prolonged periods of time, regardless of the presence or absence of *H. Pylori* in the patient).

Current known non-invasive methods are 1) $^{13}$C urea breath test ($^{13}$C UBT), which is considered the gold standard breath test; and 2) a stool test for the *H. Pylori* antigen. Their advantages are: a) the non-invasive nature of these methods, and b) the high sensitivity (95%) and specificity (95%) of the conventional $^{13}$C UBT. The disadvantages of this known $^{13}$C UBT are: a) the high cost of the equipment ($25,000 Otsuka America POCone infrared spectrometer) that requires operation by professionals who charge a fee; b) the high cost of [13]C labeled urea; c) the relatively high cost of the breath test kit ($110); and d) its very limited availability at the point-of-care and directly to the consumer as an over-the-counter product. The stool test for the *H. Pylori* antigen is a) messy; and b) of rather low sensitivity (85%) and specificity (85%). The disadvantages of both invasive and non-invasive diagnostic methods have significantly limited the detection and subsequent eradication of *H. Pylori* worldwide.

The $^{13}$C UBT is based on the ability of *H. Pylori* to hydrolyze $^{13}$C labeled urea ([13]$CO(NH_2)_2$) catalyzed by its abundant enzyme urease and produce [13]$CO_2$ and ammonia ($NH_3$) in breath as follows:

$$^{13}CO(NH_2)_2 + H_2O \xrightarrow{\textit{-urease}} {}^{13}CO_2 + 2NH_3$$

The conventional $^{13}$C UBT is supplied as a kit which contains a $^{13}$C-labeled urea meal (pranactin citric drug powder), one bag for the baseline fasting breath sample, and a second bag for the post-($^{13}$C-labeled urea) meal breath sample. The bags get transported to a centralized laboratory or are tested at the health provider's office by trained personnel, if they have the required equipment. This conventional $^{13}$C UBT measures the ratio of [13]$CO_2$/[12]$CO_2$ in the breath of individuals post-ingestion of $^{13}$C-labeled urea. This is delta over a baseline fasting breath $CO_2$. Use of $^{13}$C-labeled urea (instead of unlabeled urea) is required in order to demonstrate that the produced [13]$CO_2$ in breath comes solely from the breakdown of the $^{13}$C-labeled urea by *H. Pylori*, and not from other sources within the body which release $CO_2$ (e.g., muscles). Although $^{13}$C, a natural isotope of carbon, is encountered in nature, it is only 1.8% of the total carbon. Therefore, the chance that [13]$CO_2$ is a product of other than *H. Pylori* urea hydrolysis is infinitesimal.

The procedure for the $^{13}C$ UBT described above is as follows: a) the individual abstains from antibiotics, PPIs, bismuth, and sucralfate for two weeks; b) on the day of the test, the individual fasts (nothing by mouth) for one hour; c) the individual exhales into a bag provided by the supplier of the $^{13}C$ UBT; d) the individual ingests a $^{13}C$ urea meal; e) 20 minutes post ingestion, the individual exhales into a bag provided by the supplier; f) the professional health care provider collects the bags and runs them through a spectrometer if one is available on-site or sends them to a centralized laboratory for testing; and g) the individual is notified about the results within 48 hours when the bags are sent to the centralized laboratory or within a few minutes when the spectrometer is available on-site.

As set forth in the present disclosure, it would be desirable to provide a hand-held breathalyzer device that is easy-to-operate by non-specialized professionals; that will perform a novel urea breath test (UBT) by measuring both $NH_3$ and $CO_2$ (e.g., simultaneously) in a single breath sample; and that is inexpensive (e.g., $40-$60 per device plus test). It would also be desirable to provide such a device that would provide the results instantaneously at the point-of-care and directly to the consumer via over-the-counter (OTC) or online sale. Still further, it would be desirable to provide such a breath test that is able to utilize either $^{13}C$-labeled urea, or inexpensive (e.g., less than $1/per test) unlabeled urea.

## SUMMARY OF THE INVENTION

The invention is defined in the appended claims. Certain embodiments of the present disclosure provide a breath analyzer. The breath analyzer includes an input, a first sensor, and a second sensor. The input is configured to receive a breath sample, and the first sensor and the second sensor are each configured to contact the breath sample. In some embodiments, the breath analyzer has two separate channels. In such cases, the breath sample is configured to travel from the input, through the separate channels, to each of the first and second sensors. The first sensor includes a first conductive polymer and a conductive material. The first conductive polymer contacts the conductive material and has a resistivity that increases in response to increased concentration of ammonia. The second sensor includes a second conductive polymer and a conductive material. In some embodiments, the conductive material of the first sensor and/or the second sensor comprises a plurality of electrodes. In some cases, the plurality of electrodes comprise wires arranged in a spiral configuration. In other embodiments, the plurality of electrodes comprise wires arranged in a rectangular configuration. The second conductive polymer contacts the conductive material and has a resistivity that increases in response to increased concentration of $^{12}CO_2$ and $^{13}CO_2$. The second conductive polymer comprises sulfonated polyaniline blended with polyethylene oxide. In some cases, a ratio of polyethylene oxide to sulfonated polyaniline is from 10%-30% by weight (e.g., about 30% by weight). In certain embodiments, the sulfonated polyaniline is synthesized from an emeraldine form of polyaniline polymer. The breath analyzer further includes a processor and an electrical circuit that operably connects the first and second sensors to the processor. The processor detects resistivity in the electrical circuit and uses the resistivity to calculate a total concentration of ammonia and a total concentration of $^{12}CO_2$ and $^{13}CO_2$ in the breath sample. In some embodiments, the breath analyzer is in wireless communication with a remote device.

Other embodiments of the present disclosure provide a breath test method that includes the step or providing a breath analyzer (which can be any breath analyzer described in this disclosure, e.g., including the breath analyzer described in the preceding paragraph). The method further includes prompting the subject to exhale a baseline breath sample into the breath analyzer, allowing the processor to measure a resistivity of the first sensor that occurs when the baseline breath sample contacts the first sensor, and allowing the processor to measure a resistivity of the second sensor that occurs when the baseline breath sample contacts the second sensor. The method also includes providing the subject with a meal or capsule containing urea (wherein the urea is either $^{13}C$-labeled or unlabeled), and then prompting the subject to exhale a post-urea ingestion breath sample into the breath analyzer. The method additionally includes allowing the processor to measure a resistivity of the first sensor that occurs when the post-urea breath sample contacts the first sensor, allowing the processor to measure a resistivity of the second sensor that occurs when the post-urea breath sample contacts the second sensor, and comparing the measured resistivity of the baseline breath sample to the measured resistivity of the post-urea breath sample. The method further includes calculating the difference between the resistivity of the first sensor of the baseline breath sample and the measured resistivity of the first sensor of the post-urea breath sample, and calculating the difference between the resistivity of the second sensor of the baseline breath sample and the measured resistivity of the second sensor of the post-urea breath sample. Still further, the method includes expressing the difference in resistivity of the first sensor (e.g., in ppb of $NH_3$) and the difference in resistivity of the second sensor (e.g., in ppm of $CO_2$ or $^{13}CO_2$), and then displaying a final result as positive for *H. Pylori* when the difference between post-urea and baseline measured resistivity of the first and second sensors is a positive number and as negative for *H. Pylori* when the difference between post-urea and baseline resistivity of the first and second sensors is zero or a negative number.

Certain other embodiments of the present disclosure provide a method of detecting *H. Pylori* in a digestive tract of a subject. The method includes collecting a baseline breath sample from a subject using a breath analyzer, and determining a total amount of ammonia and a total amount of $^{12}CO_2$ and $^{13}CO_2$ present in the baseline breath sample using the breath analyzer. The method further includes allowing the subject to ingest a meal containing a predetermined amount of natural or labeled urea. The method additionally includes collecting a post-urea ingestion breath sample from the subject using the breath analyzer, and determining a total amount of ammonia and a total amount of $^{12}CO_2$ and $^{13}CO_2$ present in the post-urea ingestion breath sample using the breath analyzer. The method further includes the step of designating a presence of *H. Pylori* in the digestive tract of the subject if the total amount of ammonia and the total amount of $^{12}CO_2$ and $^{13}CO_2$ present in the post-urea ingestion breath sample exceeds the total amount of ammonia and the total amount of $^{12}CO_2$ and $^{13}CO_2$ present in the baseline breath sample by a predetermined value. The breath analyzer used in this method can be any breath analyzer described in the present disclosure (e.g., including the breath analyzer described above wherein the second conductive polymer comprises sulfonated polyaniline blended with polyethylene oxide).

## BRIEF DESCRIPTION OF THE DRAWINGS

Figure 1 illustrates an acid-base (emeraldine salt (ES)-emeraldine base (EB)) transition for polyaniline.

Figure 2 shows an embodiment of a PANI-CSA gas sensor.

Figure 3 is a graph illustrating changes in sensor conductivity for one embodiment of an $NH_3$ sensor.

Figure 4 is a graph illustrating change in current based on the changing conductivity of the sensor of Fig. 3.

Figure 5 shows a DropSens flow cell setup.

Figure 6 is a calibration plot for $CO_2$, showing change in conductivity of an $NH_3$ sensor of the present disclosure based on change in percent of $CO_2$.

Figure 7 is a graph showing sensitivities of a PANI-CSA sensor of the present disclosure to particular gases.

Figure 8 shows a $CO_2$ sensor having PPY-DBSA coated on a gold finger electrode.

Figure 9 is a graph illustrating how current to a PPY-DBSA sensor of the present disclosure changes upon exposure to $CO_2$.

Figure 10 shows calibration of a PPY-DBSA sensor of the present disclosure.

Figure 11 shows the effect of $CO_2$, $O_2$, $H_2O$, and air on a PPY-DBSA sensor of the present disclosure.

Figure 12 shows a bench prototype schematic of an embodiment of a breath analyzer of the present disclosure that includes both a PPY-DBSA sensor and a PANI-CSA sensor.

Figure 13 illustrates a diverting valve for use in a multi-sensor system of the present disclosure.

Figure 14 is a schematic illustration of a diverting valve for use in a multi-sensor system of the present disclosure.

Figure 15 shows sensor contact pads of the present disclosure connected to an analyzer through clips and copper wire.

Figure 16 shows one embodiment of a portable breathalyzer of the present disclosure.

Figure 17 shows an embodiment of a filmed finger electrode for a gas sensor of the present disclosure.

Figure 18 shows a fabrication process for an interdigitated electrode of a sensor of the present disclosure.

Figures 19a-c show various possible electrode layouts for sensors of the present disclosure.

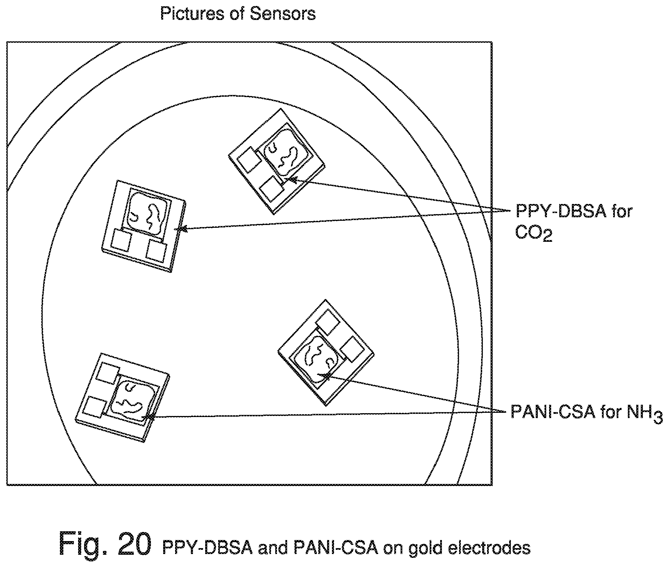

Figure 20 shows both a PPY-DBSA sensor and a PANI-CSA sensor in accordance with certain embodiments of the present disclosure.

Figure 21 shows the effect of 5% $CO_2$ on a PPY sensor doped with different dopants (i.e., PPY-ASA, PPY-HBSA, and PPY-DBSA).

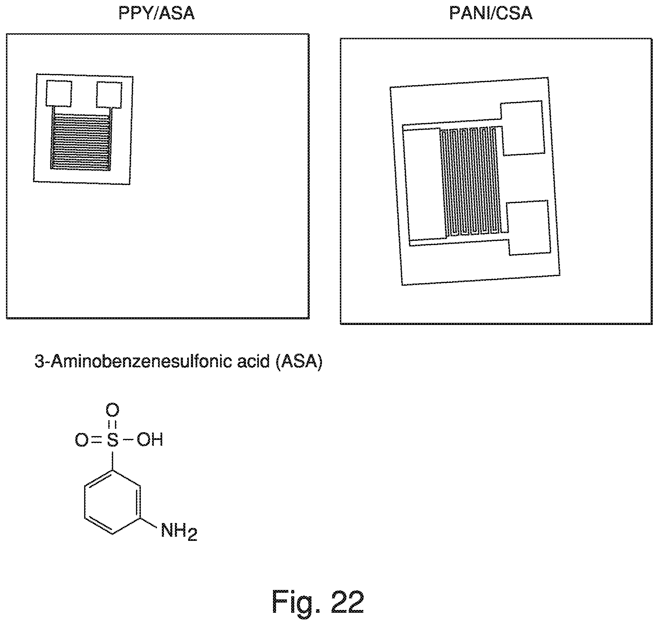

Figure 22 shows a PPY-ASA sensor and a PANI-CSA sensor in accordance with certain embodiments of the present disclosure, and also shows the chemical structure of 3-Aminobenzenesulfonic acid.

Figure 23 shows the stages of an electrochemical polymerization technique in accordance with certain embodiments of the present disclosure.

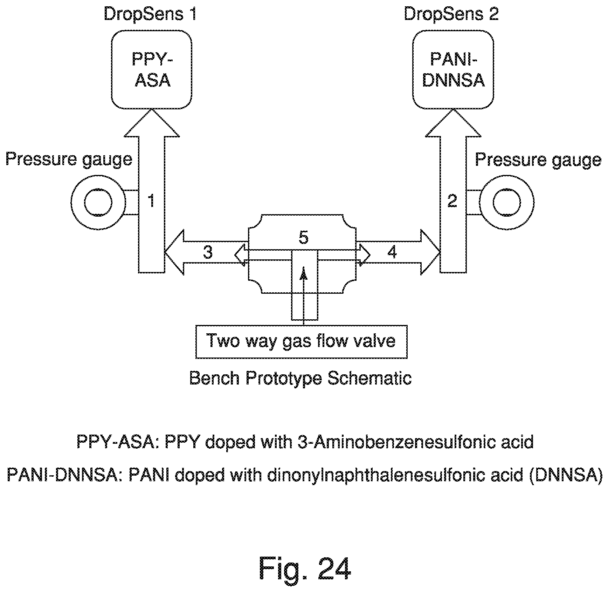

Figure 24 shows another embodiment of a breath analyzer of the present disclosure that includes both a PPY-ASA sensor and a PANI-DNNSA sensor.

Figure 25 is a photograph of an electrochemical sensor prepared by a SPANI/PEO blended solution.

Figure 26 is graph showing the conductivity measurement of SPANI/PEO (30wt%) when exposed to carbon dioxide.

Figure 27 is a graph illustrating the resistance change of different conducting polymers to $CO_2$.

Figure 28 is a graph showing $CO_2$ readings from a $CO_2$ meter sensor.

Figure 29 is a partially broken-away schematic perspective view of a bench prototype of the present disclosure.

Figure 30 is a photograph showing a bench prototype of the present disclosure.

Figure 31 is a graph showing results from a breath test using the $CO_2$ meter.

Figure 32 is a photograph showing two $CO_2$ sensors, including a PANI/ABSA sensor and a PPY/ABSA sensor.

Figure 33 is a graph showing the $CO_2$ response of SPANI with 10 wt% PEO.

Figure 34 shows a non-limiting example of a wireless communication breathalyzer device of the present disclosure.

## DETAILED DESCRIPTION OF PREFERRED EMBODIMENTS

The following detailed description is to be read with reference to the drawings, in which like elements in different drawings have like reference numerals. The drawings, which are not necessarily to scale, depict selected embodiments and are not intended to limit the scope of the invention. Skilled artisans will recognize that the examples provided herein have many useful alternatives.

Conducting polymers are widely used in gas sensor applications because they provide a stable and porous matrix for the gas component and also alter the electron transfer process. Recently, many efforts have been aimed at improving the physical properties (e.g., solubility, dispersion properties, stability, and mechanical integrity) of conductive polymers. Such composite formation is being optimized to prepare material which can be processed for sensor fabrication. The poly-conjugated conducting polymer composites have recently been used for gas-sensing applications due to several useful features like direct and easy deposition on the sensor electrode, control of thickness, and redox conductivity of polyelectrolyte characteristics. These possibilities, as well as the improvements in atmospheric stability of polypyrrole composites, make them serious candidates for use in specific technological applications. The advantages of conducting polymer composites applicability to gas sensors are their low cost, affinity for various substrates, high sensitivity, and processibility.

Although small amounts of $NH_3$ and $CO_2$ in breath can be produced under circumstances unrelated to H. Pylori infection, the simultaneous elevation of the gases $NH_3$ and $CO_2$ (and/or $^{13}CO_2$) in breath, shortly (i.e., within 10-90 minutes) after ingestion of either $^{13}C$-labeled or unlabeled urea as delta over baseline pre-urea ($^{13}C$-labeled or unlabeled) ingestion gases, guarantees that the source for these gases is solely the hydrolysis of urea ($^{13}C$-labeled or unlabeled) by H. Pylori. As described herein, the co-inventors of the present application have identified and optimized polymer-based sensors sensitive to $NH_3$, $CO_2$, and $^{13}CO_2$ that can be interfaced within a portable hand-held device that will measure $NH_3$ and $CO_2$ (and/or $^{13}CO_2$) and provide test results instantaneously. In many cases, elevation of these gases ($NH_3$, $CO_2$, and $^{13}CO_2$) can be detected no earlier than 10 minutes post ingestion and no later than 90 minutes post ingestion. Thus, the individual should exhale into the present breath analyzer device, or the individual's breath sample should be taken (i.e., collected) within 10-90 minutes (e.g., 20 minutes) after the individual ingests the urea ($^{13}C$-labeled or unlabeled) substrate.

The present invention provides an improved breath analyzer and breath test method to determine the presence of H. Pylori in a subject's digestive tract. The improved breath analyzer and breath test is less costly, more convenient, and more diagnostically accurate than existing methods and devices.

As described in greater detail below, innovative features of the breath analyzer (or breathalyzer device) of the present invention include, but are not limited to, the following: a) the identification and optimization of polymer-based sensors for the simultaneous measurement of $NH_3$, $CO_2$, and $^{13}CO_2$ at very low concentrations such as those contained in human breath; b) the interface of the signals from two sensors when a gas sample is advanced through both sensors simultaneously; c) the interface of two independent sensors; d) the software to analyze two or three different gas signals simultaneously; and e) the electronic circuit and display of results. The display can show results that are either numerical or binary (e.g., positive-negative).

As discussed above, the novel breathalyzer device of the present invention simultaneously measures the content of $NH_3$, $CO_2$, and $^{13}CO_2$ in human breath. This breathalyzer device includes two sensors, one sensitive to $NH_3$, and another sensitive to $CO_2$ and to $^{13}CO_2$. As used herein, $CO_2$ refers to $^{12}CO_2$ (rather than to $^{13}CO_2$). The molecular weights of $^{12}CO_2$ and $^{13}CO_2$ are very similar.

Although the term "simultaneously" is used throughout this disclosure when referring to measuring the content of $NH_3$, $CO_2$, and $^{13}CO_2$ in a breath sample, skilled artisans will appreciate that measurements of such gases need not occur at the exact same time. Instead, in any or all embodiments of the present disclosure, the breath sample can contact each sensor of the present disclosure at the same or approximately the same time. For example, the breath sample will not travel to the first sensor and afterward to the second sensor when using the presently disclosed method and device. Rather, in certain embodiments of the present disclosure, the breath sample will travel to both sensors through equidistant (but separate) channels from the mouthpiece to the sensors such that the breath sample reaches both sensors at the same time.

Urea breath test for the detection of H. Pylori is based on the ability the bacterium (H. Pylori) possesses to convert urea to $NH_3$ and $CO_2$. In one embodiment, the urea breath test ("UBT") of the present disclosure utilizes unlabeled urea as the exogenous substrate for the urease of H. Pylori. This UBT method measures $NH_3$ at the desired range of 25 ppb-1 ppm, and can measure $CO_2$ (alone or in combination with $^{13}CO_2$) within ranges of 14,000-28,000 ppm or 20,000-50,000 ppm, for example.

The UBT method and breath analyzer of the present disclosure detect an increase in $CO_2$, $^{13}CO_2$, and $NH_3$ gas levels in certain individuals after those individuals have ingested urea (either $^{13}C$-labeled or unlabeled). In particular, the $CO_2$ sensor of the present invention is sensitive enough to detect an increase in concentration of $^{12}CO_2$ or $^{13}CO_2$ in a breath sample from baseline from 100-1000ppm. The present breath test method includes a step of diagnosing a subject as having H. Pylori when the subject's breath sample shows such an increase in concentration of $^{13}CO_2$ or $CO_2$ from

baseline. None of $NH_3$, $CO_2$, or $^{13}CO_2$ gas, on its own, can be attributed solely to *H. Pylori* hydrolysis of urea. For example, $NH_3$ can be produced in kidney and metabolic diseases, whereas $CO_2$ can be produced from muscle breakdown and from large bowel microbes. However, the co-inventors of the present application have discovered that the simultaneous rising of both gases ($NH_3$, $CO_2$) shortly after ingestion of unlabeled urea (e.g., within 10-90 minutes after ingestion) guarantees that the source of elevation of both gases is *H. Pylori* hydrolysis of urea. When the value of post urea $NH_3$, $CO_2$, and/or $^{13}CO_2$ is higher than the baseline $NH_3$, $CO_2$, and/or $^{13}CO_2$ by a certain predetermined percentage, the UBT is positive for *H. Pylori* infection. When the value of post urea $NH_3$, $CO_2$, and/or $^{13}CO_2$ is the same as the baseline or lower than the baseline $NH_3$, $CO_2$, and/or $^{13}CO_2$, the UBT is negative for *H. Pylori* infection.

In one embodiment, the procedure for the UBT of the present disclosure is as follows: a) the individual will abstain from antibiotics, PPIs, bismuth, and sucralfate for two weeks (as they can suppress *H. Pylori*); b) prior to the UBT, the individual fasts (nothing by mouth) for one hour; c) the individual exhales into the input (e.g., mouthpiece) of the breathalyzer device once (no bags needed for the collection of breath) and this is the baseline breath sample; d) the individual ingests a small solid or liquid meal (or capsule) containing either $^{13}C$-labeled or unlabeled (natural) urea; e) within 10-90 minutes post ingestion, the individual exhales again into the input (e.g., mouthpiece) of the breathalyzer once (no bag either) and this is the post-urea breath sample; and f) the result, which is calculated by the software of the device as the difference between the post-urea ingestion and the baseline values of $NH_3$, $CO_2$, and/or $^{13}CO_2$ (i.e., post-urea minus baseline), is displayed on the display screen of the breathalyzer immediately after the second exhalation.

$NH_3$ Sensor: Polyaniline (PANI), which was first reported in 1862, is one of the most investigated and widely used conducting polymers. This is in part because it is an inexpensive monomer, and also due to its facile synthesis in acidic aqueous solutions, environmental stability, good processability, and solubility in common organic solvents (thus allowing it to be blended with other polymers).

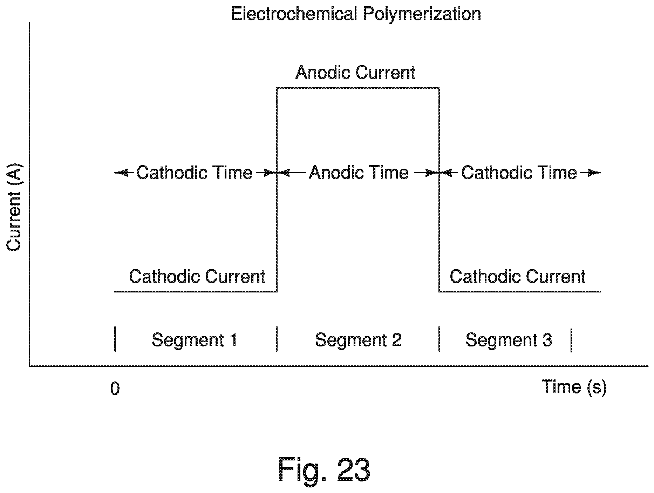

Electrochemical Polymerization of Conducting Polymers: The following five paragraphs refer to an electrochemical process that can be used to develop a PANI sensor (e.g., the PANI sensor of the present disclosure), or any electrochemical sensor. This process is illustrated in Fig. 23.

Generally, experimental set-up for electrochemical synthesis of electroconducting polymers in laboratory conditions is relatively straightforward. It involves, in many instances, a standard three-electrode electrochemical cell, although a two electrode cell can be used in certain cases of galvanostatic polymerization (Wallace et al., 2009). The polymer obtained by this procedure is deposited directly on the electrode. The anodic and cathodic electrolytes can be passed through electrode compartments at specified flow, while polymerization can be achieved at constant potential. The most common experimental techniques used for electrochemical polymerization of aniline are: cyclic voltammetry (potentiodynamic), chronopotentiometric, galvanostatic, and potentiostatic techniques.

Polymerization using chronopotentiometry is characterized by cyclic regular change of the electrode potential and the deposited polymer is, throughout the experiment, changing between its non-conducting and conducting (doped) state, followed by exchange of the electrolyte through polymer (Heinze et al., 2010). At the end of polymerization, the obtained polymer is in its non-conducting form. Relatively high potential is required for electrochemical oxidation of aniline monomer. Therefore during the first 2 - 10 cycles, the upper potential limit is high, but as a result of the autocatalytic nature of aniline electropolymerization, the upper potential limit can be decreased to avoid degradation that results from over-oxidation of the pernigraniline form of polyaniline (Inzelt, 2008).

Electrochemical polymerization of aniline proceeds together with insertion of chloride anions (dopant) from the electrolyte, according to the following equation:

$$(PANI)_n + nyCl^- \rightarrow [PANI^{y+}(Cl^-)]_n + nye^-$$

Where y refers to doping degree, ratio between the number of charges in the polymer and the number of monomer units (Kankare, 1998).

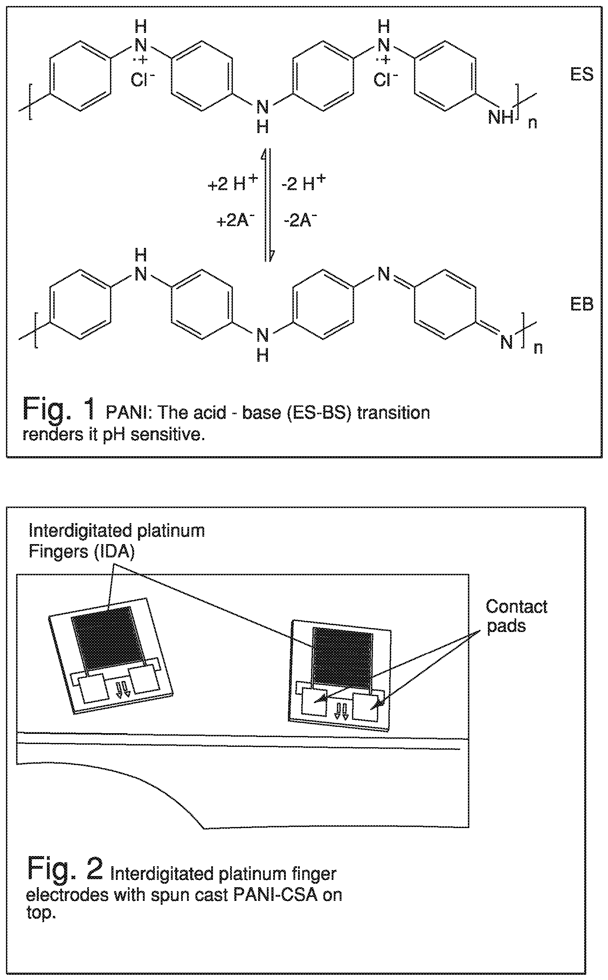

PANI exhibits three different oxidation states: leucoemeraldine (LEB, fully reduced), emeraldine (EB, half-oxidized) and pernigraniline (PNB, fully oxidized). However, emeraldine salt, the protonated form of EB, is the only conducting form and is usually obtained by protonation of the basic amine and imine sites in EB with strong acids. This is an important characteristic of PANI, especially for ammonia detection, as it deprotonates the amine groups in the emeraldine salt, converting it to the emeraldine base form, with a corresponding drop in conductivity of several orders of magnitude. This transition of polyaniline (from the emeraldine salt (ES) to the emeraldine base (EB)) is shown in Fig. 1. The reaction that allows this change

$$PANI^+ + NH_3 \leftrightharpoons PANI + NH_4^+$$

in conductivity is as follows:

PANI doped with camphor sulfonic acid (CSA) has been shown to have a pH sensitivity of around 70mV, which is higher than the 59 mV typically observed with other small counter-ions (like Cl- and $SO_4^{2-}$). This is the basis for selecting CSA over the other protonic acids for the PANI ammonia sensors.

PANI has the feature of a flexible chain, re-doping ability, and high dispersibility in many organic or aquatic solvents (depending on the dopant). In particular, sulfonated polyaniline, explained in greater detail below, has high sensitivity to carbon dioxide with high stability and excellent conductivity.

Fabrication of $NH_3$ Sensor: A non-limiting embodiment of an $NH_3$ sensor of the present disclosure is shown in Fig. 2. To fabricate such a sensor, the following steps were performed. PANI doped with HCl was prepared by chemical oxidative polymerization of aniline in aqueous acidic medium (1M HCl) with ammonium persulfate (APS) as an oxidant. It has been previously reported that higher polymerization yields were obtained by using oxidant to monomer ratio of 1.2. The mixture was left to polymerize overnight at room temperature. The PANI precipitate was collected on filter paper and washed repeatedly with 0.1M HCl followed by repeated washes with acetone. Deprotonation of the resulting PANI salt was performed by stirring the powder in an aqueous 0.1M $NH_4OH$ solution for 24 hours at room temperature, thus obtaining the emeraldine base (EB), which was then washed with water repeatedly until neutral pH, and then dried under vacuum for 48 hours at 60°C. PANI was re-doped with CSA with a molar ratio of 2:1. Thin Film interdigitated platinum electrodes (IDA) (line spacing 100$\mu$m) were purchased from the Electronic Design Center, Case Western University. The films were prepared by spin coating the PANI-CSA solution on the IDA electrodes. Prior to spin coating, the electrodes were cleaned by rinsing in methanol followed by rinsing with deionized water and drying in a stream of dry nitrogen. PANI films were then spun cast onto the IDA (Fig. 2).

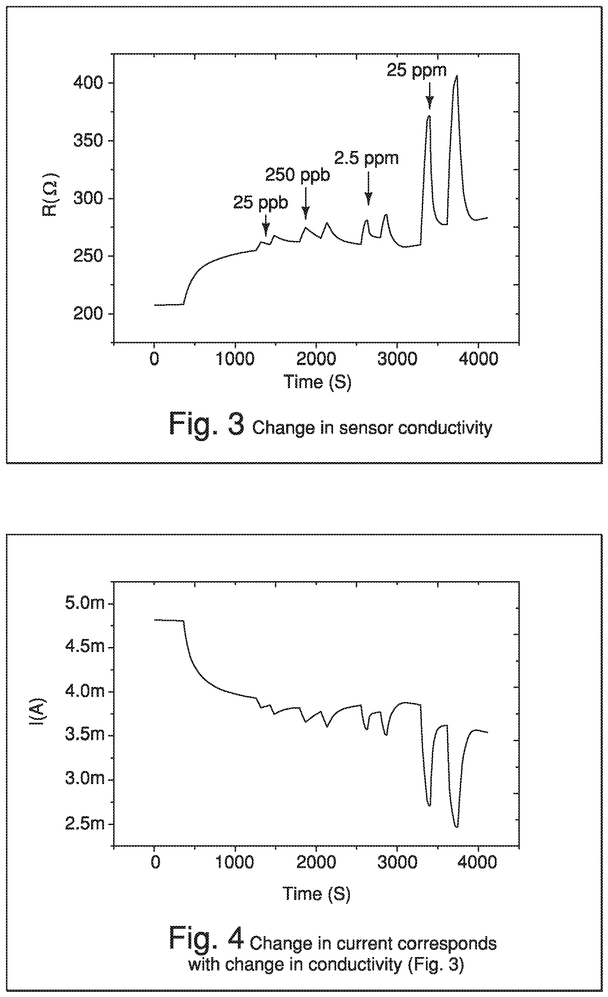

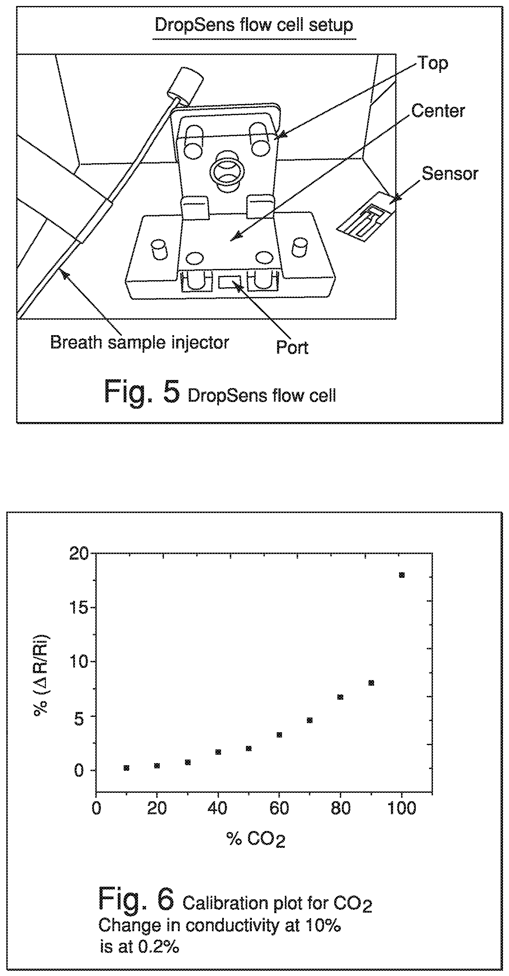

$NH_3$ sensor characterization: Calibration (Figs. 3 and 4) was performed by first making serial dilutions of the 25ppm ammonia to 2.5 ppm, 250 ppb and 25 ppb, i.e. 10-fold dilutions each in tedlar bags. A 20 cc syringe was used to collect the sample from the tedlar bags and then injected the sample directly over the sensors. A DropSens flow cell setup (Fig. 5) was used to inject ammonia gas over the sensor, which was placed in the center of the DropSens flow cell. Teflon tubing was used to connect the gas to the flow setup to minimize any $NH_3$ absorption.

Measurements were made by applying a fixed potential of 1V to the sensor (contact pads) and measuring the resulting current which changed as a function of gas passing over the sensor surface (Figs. 3 and 4). An Agilent 4155C semiconductor analyzer was used for taking the measurements, and customized test software was developed using the EasyDesktop software provided with the analyzer. Similar changes in conductivity and current were observed when using the Model 1010 gas diluter. Model 660D potentiostat analyzer (CH Instrument, Austin, TX) was used to measure sensor resistivity. The change of resistivity observed for 250 ppb and 25 ppb was around 4.8% and 2.5%, respectively, which is high enough so that breath samples in the trace level ppb values can be analyzed reliably.

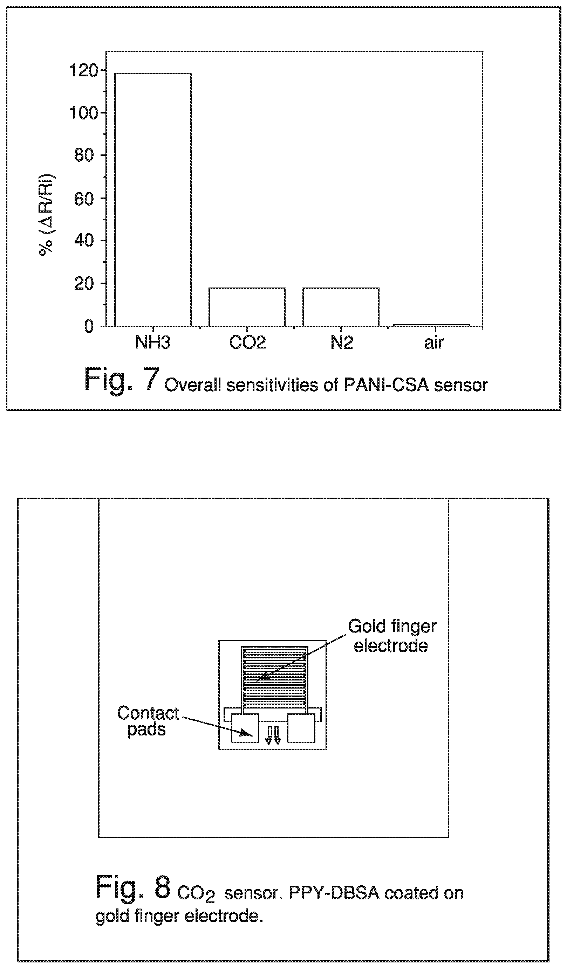

Sensitivity of the $NH_3$ sensor of the present disclosure: The human breath contains $CO_2$, $N_2$, $O_2$, $NH_3$, NO, $H_2O$, methane, volatile organic compounds (VOCs) and other gases in trace amounts. To assess the effect of breath $CO_2$ at 4-5% concentration on the PANI-CSA sensor, the co-inventors of the present application tested a range of $CO_2$ concentrations. The effect at the 4-5% $CO_2$ concentration is negligible and therefore not measurable, as demonstrated in Fig. 6. The overall sensitivity of the PANI-CSA sensor to $NH_3$, $CO_2$, $N_2$ and atmospheric air is demonstrated in Fig. 7. The PANI-CSA shows much higher response to $NH_3$ compared to $CO_2$, $N_2$, and air when taking into account that the concentration of $NH_3$ was 25 ppm (0.000025%), while 100% dry gas was used for $CO_2$ and $N_2$ (human breath contains 4-5% $CO_2$ and traces of $N_2$).

$^{13}$C is a natural isotope of carbon and is encountered in nature at a concentration of 1.8% of the total carbon. Therefore, the chance that an increase in the concentration of $^{13}CO_2$ in breath is a product of other than *H. Pylori* urea ($^{13}$C-labeled urea) hydrolysis is infinitesimal. The polymer-based $CO_2$ sensor of the present disclosure is also sensitive to $^{13}CO_2$ and can detect both $CO_2$ and $^{13}CO_2$, either individually or cumulatively. As a result, the present invention can be used for the detection of *H. Pylori* after ingestion of either $^{13}$C-labeled or unlabeled urea.

$CO_2$ sensor: Much of the work on $CO_2$ gas sensing is reported using doped or undoped $SnO_2$. Less attention has been given to electrically-conducting polymers such as polypyrrole, which has unique properties such as low density, versatility in methods of production, high anisotropy of electrical conduction, and non-metallic temperature dependence of conductivity, in regards to $CO_2$ gas sensing. Polypyrrole (PPY) can be prepared by various methods such as chemical, electrochemical, and vapor phase routes.

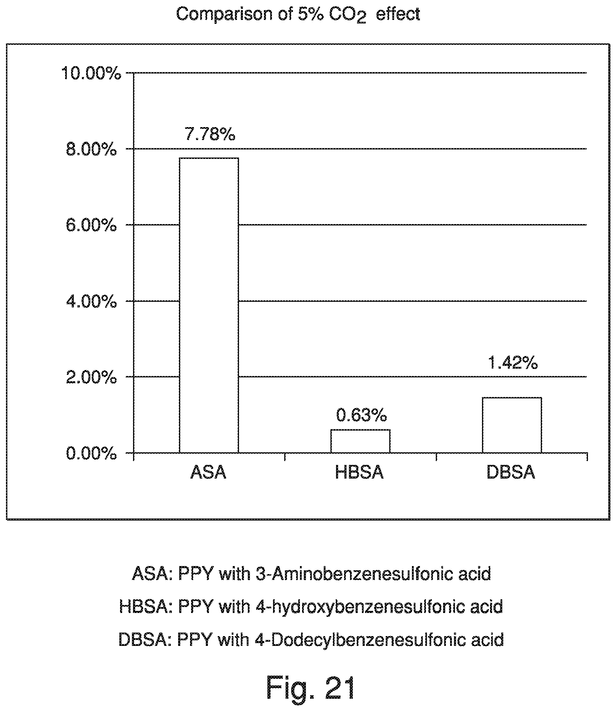

It has been reported than when polypyrrole, a type of organic polymer, gets oxidized it becomes a conducting polymer. The present co-inventors have tested the response of doped polypyrrole to 5% $CO_2$ (Figure 21). When polypyrrole is doped with 3-Aminobenzenesulfonic acid (ASA), the $CO_2$ effect is more significant than when polypyrrole is doped with 4-hydroxybenzenesulfonic acid (HBSA) or with 4-Dodecylbenzenesulfonic acid (DBSA), such that PPY-ASA is significantly more responsive to $CO_2$ than is either of PPY-DBSA or PPY-HBSA.

$CO_2$ sensor fabrication: In one embodiment of the present disclosure, a $CO_2$ sensor, particularly a PPY-based sensor, was fabricated using dodecylbenzene sulfonic acid (DBSA) as a dopant (Fig. 8). Pyrrole (Aldrich) was dried with $CaH_2$ for 24 hours, followed by distillation under reduced pressure. Ammonium persulfate (APS, Kanto Chemical Co. Inc.) was used as an oxidant and dodecylbenzene sulfonic acid (DBSA, Aldrich) was used as a dopant. 0.15 mol of DBSA and 0.3 mol of pyrrole were dissolved in 500 ml of distilled water with vigorous stirring. 0.06 mol of APS in 100 ml of distilled water was slowly added to the above solution which was maintained at a temperature of 0°C. Obtained PPY powder (1 g) was completely dissolved by ultrasonification in 25 ml of only m-cresol or chloroform with an additional 1 g of DBSA, and filtered through a 1 $\mu$m Teflon membrane filter. The solution was transferred onto Interdigitated platinum finger electrodes with gold circuit (Case Western University) and the solvent was dried, resulting in an excellent quality of free-standing film with a thickness of about 100 $\mu$m. FT-IR was used to characterize feature peak of dopant and pyrrole. Smooth and coherent films were obtained by the above preparation and cast onto an additional wafer surface for examination under a scanning electron microscope (SEM).

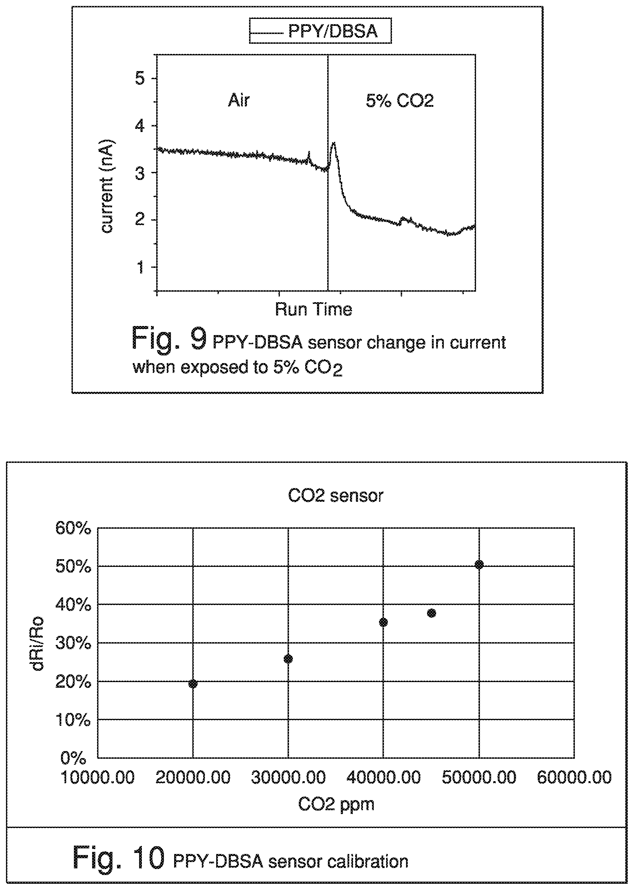

$CO_2$ Sensor Resistivity: A DropSens flow cell setup (Fig. 5) was used to examine the properties of the PPY-DBSA sensor of the present disclosure. The change in current (resistivity) of the PPY-DBSA sensor when exposed to 5% $CO_2$ (Fig. 9) was measured using Model 660D potentiostat analyzer (CH Instrument, Austin, TX) using the amperometric i-t technique. The PPY-DBSA sensor was tested for sensitivity to $CO_2$, $O_2$, $H_2O$ and atmospheric air.

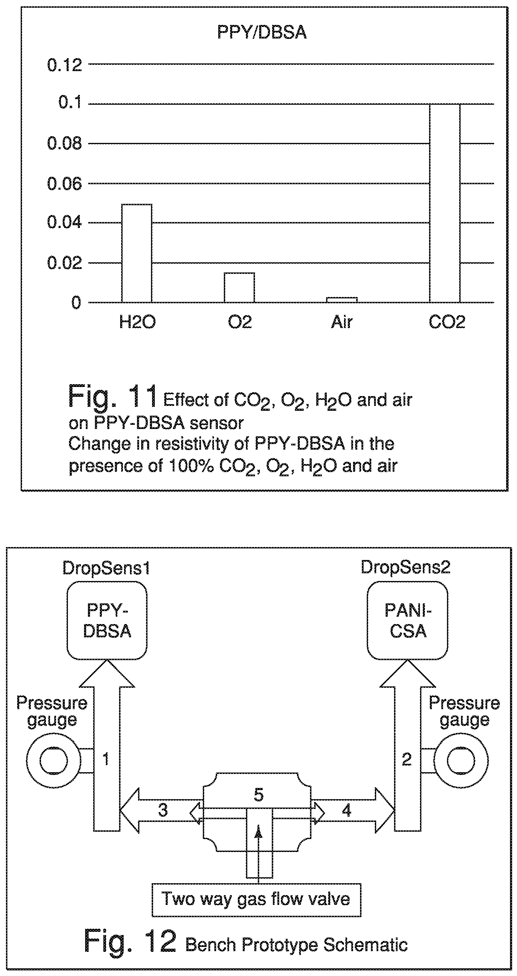

$CO_2$ Sensor Calibration: The PPY-DBSA sensor of the present invention was calibrated using 100% dry gas. Precision Gas Diluter Model 1010 from Custom Sensor Solutions was used to transport the analytic gas ($CO_2$) into the sensor in the center of the DropSens flow cell. Mass and volumetric flow meters (Omega Engineering, Inc., Norwalk, CT) with accuracy of 0.8% were connected to log the flow. The change in resistivity changed with the change in concentration of the $CO_2$ (Fig. 10). The $CO_2$ sensor was tested for its response to $O_2$, $N_2$, $H_2O$ and air (Fig. 11.) The effect of air is negligible and the effect of 100% $O_2$ gas is minimal. $H_2O$, however, has a greater response on the resistivity of the $CO_2$ sensor.

Both sensors of the present invention ($CO_2$ and $NH_3$) require optimization, as described in greater detail below below.

$NH_3$ Sensor Optimization: The co-inventors of the present application have developed the set-up to fabricate a PANI-CSA sensor and have examined its properties. Published reports have shown that patients who test positive for *H. Pylori* infection have a baseline breath concentration of $NH_3$ in the range of 0.02-0.17 ppm, and an increased concentration of $NH_3$ in breath after ingestion of unlabeled urea in the range of 0.05-1 ppm.

Effect of $CO_2$, $N_2$, $O_2$, and $H_2O$: To optimize the PANI-CSA sensor further, the PANI-CSA sensor is exposed to conditions similar to those occuring in human breath. The human breath contains mainly $CO_2$, $N_2$, $O_2$, and $H_2O$. Based on the present co-inventor's preliminary data, the sensor's response to 100% $CO_2$ and 100% $N_2$ is minimal and the response to atmospheric air is negligible. Since the human breath contains far less than 100% $CO_2$ and $N_2$, the interference from $CO_2$ and $N_2$ in breath will be negligible and therefore insignificant.

The effect of humidity on the $NH_3$ sensor: $NH_3$ is hydrophilic and the humidity of the human breath will interfere with the sensor's response to $NH_3$. To address the effect of humidity, a desiccant is used and titrated with the use of the VTI RH-200 Relative Humidity Generator.

$CO_2$ Sensor Optimization: The co-inventors of the present application have developed the set-up to fabricate a PPY-DBSA sensor highly sensitive to $CO_2$. Based on published reports about the gold standard $^{13}C$ UBT, patients infected with *H. Pylori* have elevated $^{13}CO_2$ in breath post ingestion of $^{13}C$-labeled urea, and the percentage increase in breath of $^{13}CO_2$ over the $CO_2$ baseline is between 2.4% and 4.4%, depending on the amount of ingestible urea. Based on these findings, the co-inventors of the present application believe the percentage rise in $CO_2$ over baseline after ingestion of 150-300 mg of unlabeled urea will be similar to that of the $^{13}CO_2$ after ingestion of $^{13}C$-labeled urea.

Based on the sensor calibration (Fig. 10), it is believed that the $CO_2$ sensors of the present disclosure are sensitive even below the lowest percentage rise of $CO_2$ in patients infected with *H. Pylori*. Although there are no reports that PPY-DBSA is sensitive to $NH_3$, the co-inventors of the present application will examine whether the sensor is sensitive to $NH_3$, and if so, will place a $NH_3$ specific filter (for example, 3A form crystalline metal aluminosilicates) directly over the $CO_2$ sensor to block the $NH_3$ from coming into contact with the $CO_2$ sensor. This filter, which is a desiccant, blocks both $NH_3$ and $H_2O$.

Effect of $CO_2$, $O_2$, $H_2O$, air and $NH_3$ on $CO_2$ sensor: the co-inventors of the present application have tested an embodiment of a $CO_2$ sensor of the present disclosure for response to $CO_2$, $O_2$, $H_2O$ and air (Fig. 11). Since the effect of air is negligible, it is believed that the $O_2$ and $N_2$ of human breath will only have a negligible effect, if any, on the $CO_2$ sensor since the content of $O_2$ and $N_2$ in human breath is far less than the 100% gas that the present co-inventors tested. No further examination of the effect of $O_2$ and $N_2$ on the $CO_2$ sensor is believed to be necessary.

Effect of humidity on $CO_2$ sensor: the present co-inventor's preliminary data shows that humidity affects the resistivity of the present PPY-DBSA $CO_2$ sensor (Fig. 11). To counter this effect, desiccant can be used, and the amount of desiccant can be titrated by using various amounts and examining their effect on the resistivity of the $CO_2$ sensor in the presence of the desiccant. The VTI RH-200 Relative Humidity Generator can be used to generate various levels of relative humidity (RH) and to calculate the amount of desiccant needed for the optimal level of RH which achieves optimal sensitivity (resistivity change) to $CO_2$ and/or $^{13}CO_2$.

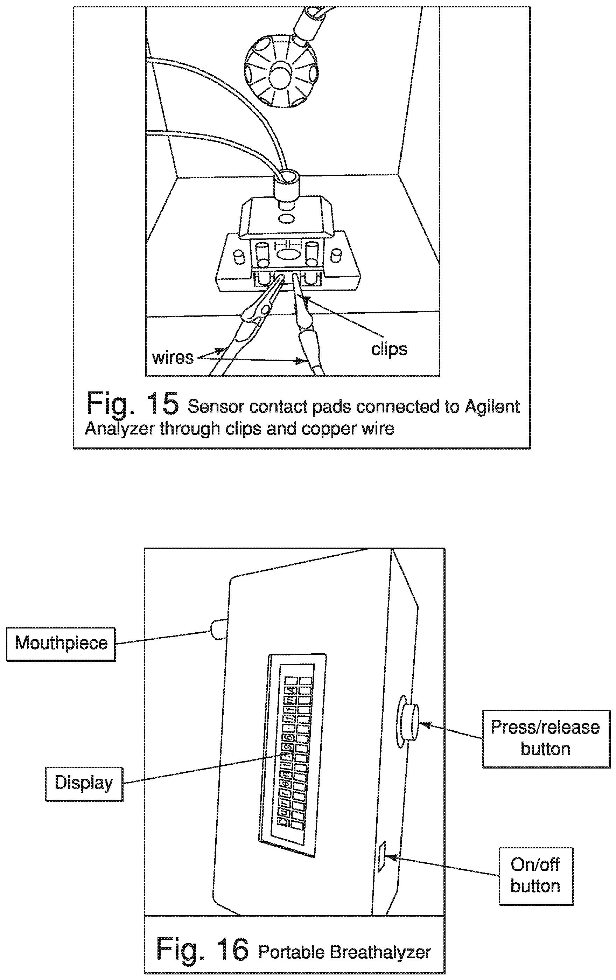

One embodiment of the portable, hand-held breathalyzer device of the present invention is shown in Fig. 15. As discussed above, this device measures both $NH_3$ and $CO_2$ (and/or $^{13}CO_2$) in breath for the detection of *H. Pylori* infection. In a bench prototype of the present device, the co-inventors of the present application will interface both sensors (e.g.,

PANI-CSA and PPY-DBSA or SPANI); will standardize the prototype for both gases ($CO_2$ and $NH_3$); and will optimize the prototype for the eventual use of breath samples from patients with *H. Pylori*.

Non-limiting embodiments of bench prototypes: In one embodiment of the present disclosure, using the fabricated and optimized PANI-CSA and PPY-DBSA sensors, a bench prototype, such as the one shown in Fig. 12, can be set up. TwoDropSens flow cells can be used, including DropSens 1 for PPY-DBSA sensor to measure $CO_2$, and DropSens 2 for the PANI-CSA sensor to measure $NH_3$. In another embodiment of the bench prototype, the DropSens flow cells are eliminated and the configuration of the bench prototype will be without flow cells. In this embodiment, the sensors will be directly connected to CH instrument for gas analysis.

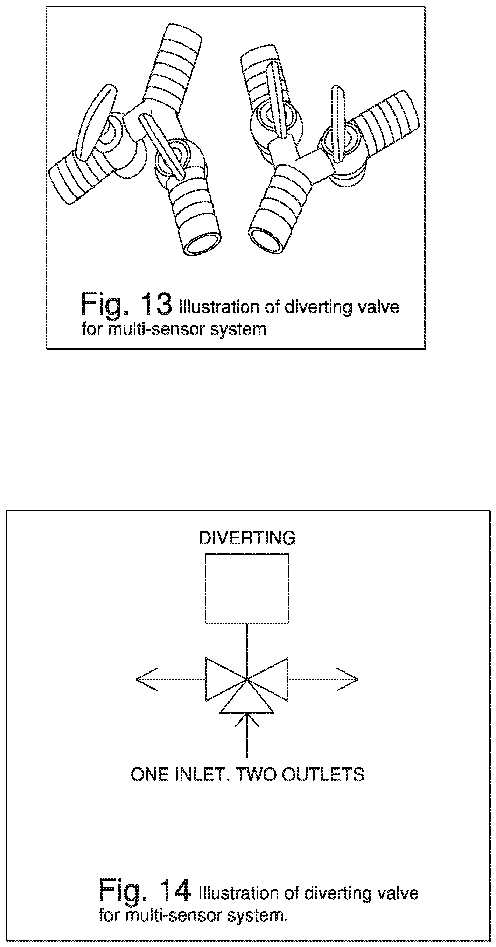

The two DropSens flow cells are connected through tubing 1, 2, 3, 4 to the two-way gas flow valve 5. A photo and schematic of the two-way valve are shown in Figs. 13 and 14, respectively. A humidity filter and/or desiccant will be placed in tubing 1 and 2 in a configuration (round, helical, etc.) that will optimize the flow of gas mixture to each DropSens. A pressure gauge will be mounted on tubing 1 and 2. The pressure gauge will regulate the flow of gas (or breath) in each tube (1, 2) so that gas flow to each sensor (DropSens 1 and DropSens 2) is the same in the presence of desiccant in each tubing. Skilled artisans will appreciate that desiccant may change the flow of gas through the tubing which flow, in turn, can be adjusted with the use of pressure gauge in tubes 1, 2.

The two-way gas flow valve will be used during optimization of the bench prototype to divert the gas mixture to each side of the device. Analytic gas is pre-treated for removal of water, airborne particles, or unwanted gas, before being characterized by each gas sensor. In the case of two gas sensors, as in the bench prototype of Fig. 12, the main gas stream will be split into two individual gas streams for pre-treatment by a diverting valve (e.g., as shown in Figs 12 and 13). The flow ratio of two outlet streams can be tuned by opening either or both of the pressure gauges.

The diversion to one or the other sensor is solely for characterization and calibration of each sensor individually. Eventually, a mixture gas containing $NH_3$ and $CO_2$ will advance to both sensors and the valve will be open to both directions. Each sensor will accept the same mixture of gases, as will be the case when an individual breathes into the device. The same breath will then go to each sensor. This explains why the $NH_3$ sensor is fabricated so that $CO_2$ has no effect on it, and why the $CO_2$ sensor is fabricated (and desiccant which blocks $NH_3$ is placed directly over it) so that $NH_3$ has no effect on it. The present co-inventors have demonstrated from experiments described herein that the remaining elements of breath (other than $CO_2$ and $NH_3$) have no effect on either sensor.

Measurement of $NH_3$ and $CO_2$: In the proposed bench set-up (Fig. 12), the sensors will be exposed to a gas mixture ($NH_3$, $CO_2$, and/or $^{13}CO_2$), as described above. The sensors' contact pads (Figs 2, 5) will be connected to the analyzer through clips and copper wires (Fig. 15). The present co-inventors will use the Agilent 4155C semiconductor analyzer and customized test software developed using the EasyDesktop software which is provided with the analyzer. The measurements of two or more gases, $NH_3$ and $CO_2$ (and/or $^{13}CO_2$), will be obtained by applying a fixed potential of 1V for 200 seconds to the sensors and measuring the resulting current change as a function of gas passed over the sensors' surface.

Approaches for Improving $CO_2$ (and $^{13}CO_2$) Affinity of the $CO_2$ Sensor:

Method 1 for fabricating the $CO_2$ Sensor: Amine modification of PPY

Amine modification of sorbents is frequently employed to introduce $CO_2$-philic functionality. In principle, the nitrile groups in PPY could be reduced to primary amines using a variety of reagents. However, reactions that work well with low molar mass compounds are not always effective when applied to high molar mass polymers.

Polypyrrole-based polymers (PPY) have been used for $CO_2$ detection, as aromatic amine is the focus for the specific chemical interaction. Although PPY shows high conductivity and sensitivity to $CO_2$, it is very difficult to work with (e.g., due to its rigid backbone and low solubility) and has poor stability characteristics. It is mainly because the aromatic five-member rings are linked covalently to each other and, especially in a doped state, have very limited rotational freedom and poor solubility. Polythiophene is also five-member ring and has similar problems. In the 1990's, others attempted to solve the issue by alkylating thiophene monomers. The polymers became soluble, even processable, in an undoped state and obtained much attention. However, after doping, the stiffness of each aromatic unit limited the solubility regardless of the alkyl groups. It will be appreciated by skilled artisans that chemical modification is a complicated synthetic process, and dispersion agents make the process less precise.

To increase amine content and thereby improve $CO_2$ (and $^{13}CO_2$) affinity of an embodiment of the $CO_2$ sensor, the present co-inventors used diamino naphthalene sulfonic acid (DANSA) as a dopant. The incorporation of primary amines enhances $CO_2$ affinity, compared to the parent polymer, as demonstrated by the present co-inventor's gas adsorption experiments on membrane samples coating on electrode, and by gas characterization studies of the membrane sensor.

Another method for fabricating a $CO_2$ sensor is to polymerize PANI and oxidize it with 3-Aminobenzenesulfonic acid. PANI is a more stable polymer than polypyrrole (PPY) and doping PANI with 3-Aminobenzenesulfonic acid (ASA) produces a sensor having a much greater response to $CO_2$ than does a PPY-ASA sensor.

Method 2 for fabricating the $CO_2$ Sensor using sulfonated polyaniline (SPANI)

A desirable polyaniline is sulfonated polyaniline ("SPANI"), which is self-doped as the sulfonate groups are covalently linked in the polyaniline backbone. SPANI is also water soluble, as the additional sulfonate groups provide water solubility (which works for gas sensing, but creates a problem in aqueous use). Water soluble poly[bis(4-phosphonicacid) phenoxy] phosphazene (PPAP) can be selected which can be a good candidate as a doping agent for PANI. Freshly prepared and dried acid functional aryloxypolyphosphazene (PPAP) was dissolved in 10 mL of water at 0 - 5 °C in a round bottomed 100mL flask and aniline monomer was added (2:1 ratios of ANI:PPAP) by vigorous stirring. Then the ammonium persulfate solution in water (aniline/oxidant ratio of 1:1) was slowly added to the reaction mixture as an oxidant. The reaction turned green in 1 hour and was allowed to stir overnight. Dark green solutions obtained from dialysis membrane were transferred into a Teflon petri dish and dried in a vacuum oven at 50 °C overnight to yield SPANI and was confirmed by FTIR and P-NMR.

Method 3 for fabricating the $CO_2$ Sensor using water-soluble sulfonated polyaniline (SPANI)

For SPANI synthesis, emeraldine hydrochloride PANI polymer powder (1 g) was dispersed in 150 ml of dichloroethane (DCE) at 80°C. The chlorosulfonic acid (1.2 ml) diluted with 2 equivalent solutions of 15 ml of DCE (i.e., 15 ml + 15 ml) were prepared and added into dispersed solution for 30 minutes, and stirred for 5 hours. In the event that the first 15 ml ran out, the 2nd 15 ml could be used until the reaction time totaled 30 minutes. Then the solid product was collected by filtration, washed carefully with cold water twice, immersed in 200 ml of distilled water, and heated for 4 hours in 100 °C to allow hydrolysis. After hydrolysis, the green solution was dried by vacuum evaporation at 40 °C. The crude SPANI powder was collected, washed by methanol for removing impurities, and then filtered and dried in a 40 °C oven.

Alternatively, SPANI aqueous solutions can be prepared by dissolution of SPANI in water. The structure can be confirmed by P-NMR and the concentration of SPANI in aqueous solutions can be measured by means of UV-vis-spectroscopy. Other solvents (i.e., other than DCE and water) can be used to dissolve the SPANI, so long as the solvent is able to dissolve PANI and acid.

Sulfonated polyaniline (particularly when prepared by the above-described Method 3) shows good solubility in water. However, the low evaporation and hydrophobicity of the finger electrodes are the result of poor adhesion with the electrodes and the polymer. To address such issues and increase adhesiveness of the film, polyethylene glycol ("PEG") or polyethene oxide ("PEO") was used as a binder due to its low glass transition temperature ($T_g$) and hydrophilic properties.

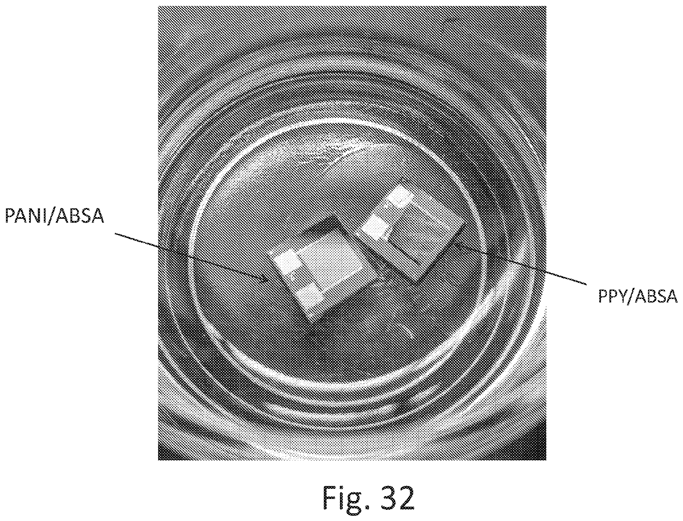

Alternative methods to increase adhesiveness of the film can include using electrochemical polymerization, or a PPY/ABSA film (4-acetamidobenzenesulfonyl azide) (i.e., polypyrrole doped with with ABSA). Two example $CO_2$ sensors are shown in FIG. 32, including a PPY/ABSA sensor, and a PANI/ABSA sensor (i.e., polyaniline doped with ABSA). Applicant discovered that electrical conductivity of the PANI/ABSA sensor is low (~1E-12)

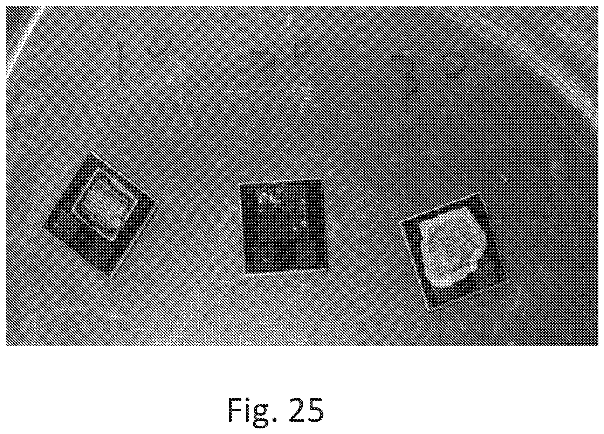

As shown in FIG. 25, three different mass ratios (10 wt%, 20%, and 30%) of PEO were blended with SPANI and dropcasted on finger electrodes. However, for the low ratio of PEO (10 wt%), little phase separation was observed. However, for the high ratio of PEO (30 wt%), the membrane has a uniform and one-phase appearance. The baseline conductivity in the air was 60 +- 5 $\mu$A for 0.5V.

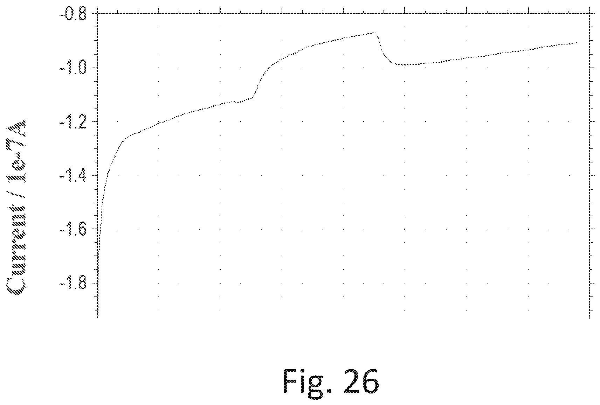

The conductivity measurement was carried by CH Instrument by applying 0.5V and measuring the appearing current. As shown in FIG. 26, baseline (air) shows a stable value and low noise. The current change due to 5% $CO_2$ is shown, and baseline could be recovered in a very short time when purging with air.

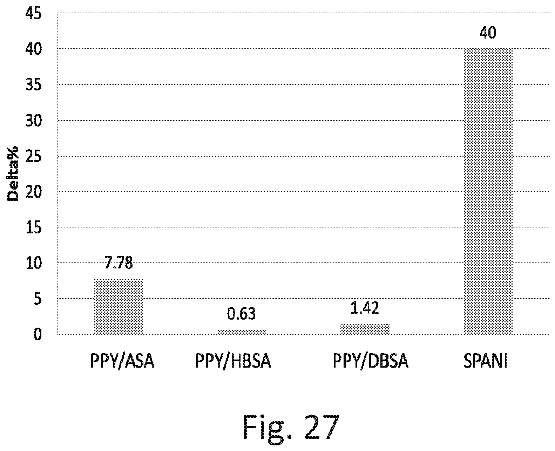

Four different conducting polymer electrochemical sensors were prepared and tested for their response to $CO_2$. The conducting polymers tested included PPY/ASA; PPY/HBSA; PPY/DBSA; and SPANI. The test gas was 5% carbon dioxide. When the SPANI sensor was tested, the resistance change ratio was 40% after exposure to carbon dioxide. These results are shown in FIG. 27 and demonstrate that SPANI has high potential for $CO_2$ sensing applications.

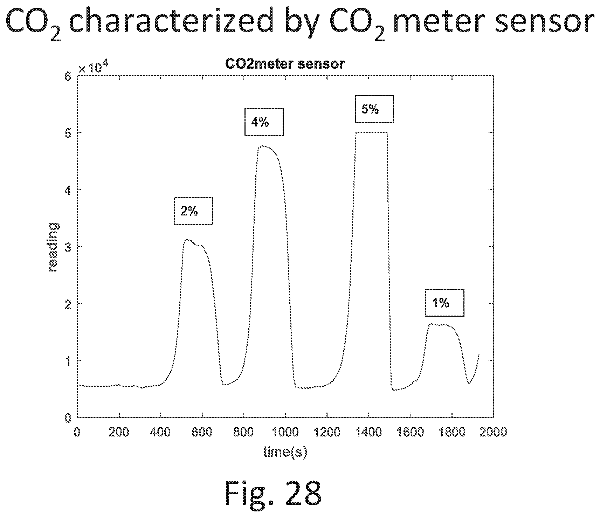

Figure 28 is a graph showing $CO_2$ readings from a $CO_2$ meter sensor. The working range of the $CO_2$ meter is below 5% and the sensitivity is at or about 100ppm.

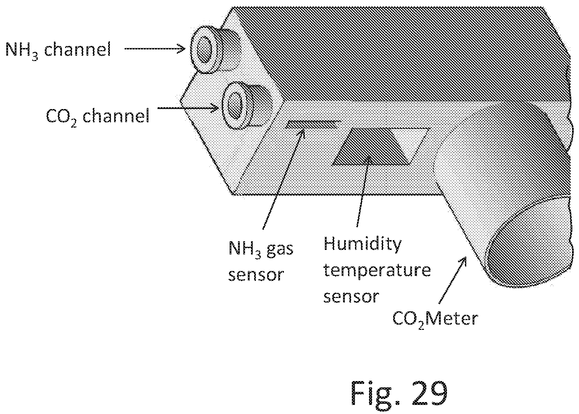

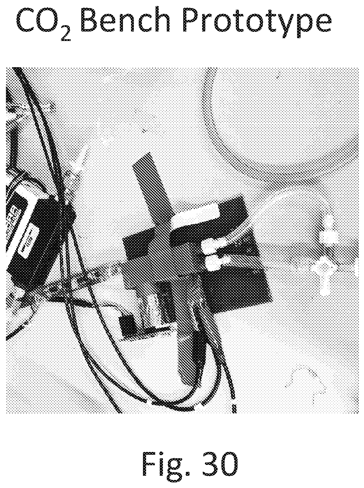

Figures 29 and 30 show bench prototypes. As illustrated schematically in FIG. 29, the prototype has dual channels for $CO_2$ and $NH_3$ gas characterization. In particular, the prototype includes an $NH_3$ sensor, an $NH_3$ channel, a $CO_2$ channel, a $CO_2$ meter, and a temperature and humidity sensor. Ammonia and $CO_2$ channels are configured to accept the breath sample through a tube (not depicted) which serves as a mouthpiece. As shown in FIG. 30, the $NH_3$ sensor location indicates the space where the ammonia sensor is placed in the prototype. Skilled artisans will appreciate that the location of the ammonia sensor may be different from that shown in FIG. 30. The $CO_2$ meter of the bench prototype is an industrial type of $CO_2$ sensor (ExplorIR-M 5% $CO_2$ sensor) that was used to study the prototype device.

The bench prototype also includes a humidity and temperature sensor, one possible location of which is shown in FIG. 30. In most cases, the temperature and humidity sensor will indicate that the temperature is room temperature (e.g.,

around 70 degrees F). The humidity is expected to be different for the $NH_3$ sensor and the $CO_2$ sensor. This is because humidity of the breath will in most cases be blocked at about 80-90% for the $NH_3$ sensor (e.g., by using a desiccant). In some embodiments, a desiccant will be used in the $CO_2$ channel to block humidity of the breath sample to a certain degree prior to the breath sample reaching the $CO_2$ sensor.

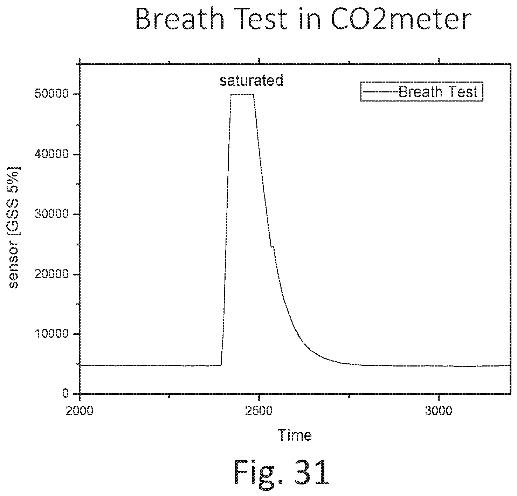

Figure 31 is a graph showing results from a breath test using the $CO_2$ meter. As shown, the signal of the $CO_2$ meter becomes saturated with breath, and the working range is 0-5% $CO_2$.

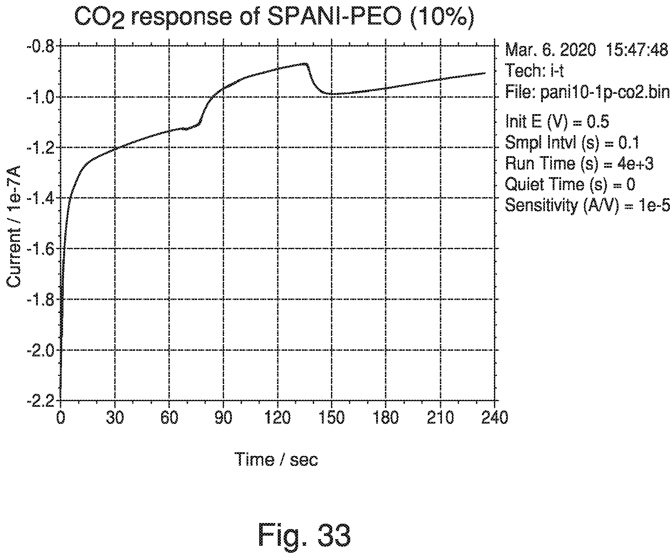

Figure 33 is a graph showing the $CO_2$ response of SPANI with 10 wt% PEO. As shown, with the SPANI-PEO (10%) sensor, noise is reduced much more (as compared to PANI/ABSA). and the change in current (I%) for 1% $CO_2$ was 13%.

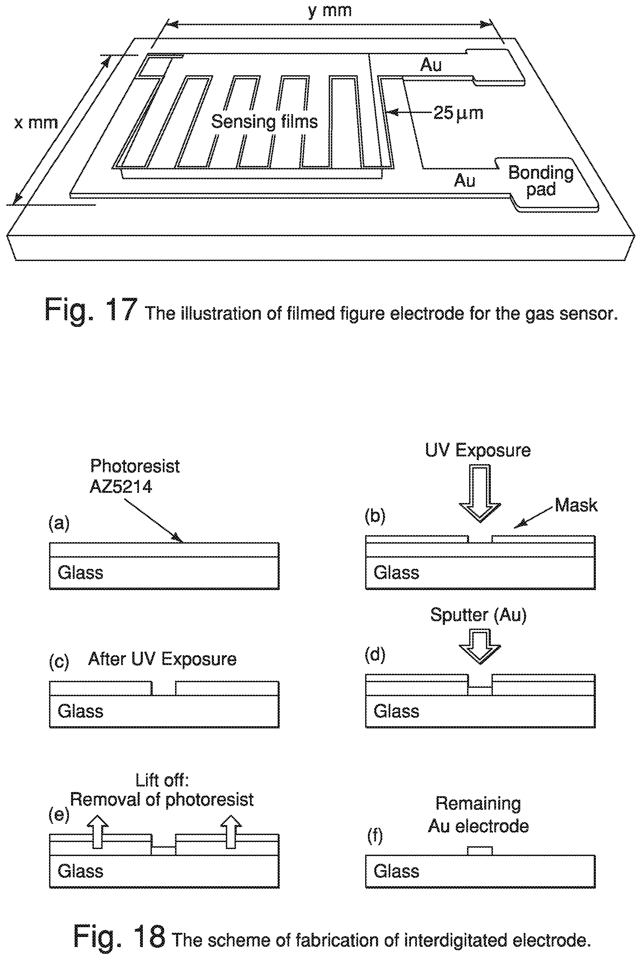

Conductivity measurements and the closely related salinity measurements are important in a number of applications from specific gas characterization and sensing. A typical layout of a finger electrode in the area of gas sensors is shown in Figure 17. Extensive work has been explored on $H_2$, $O_2$, $NO_x$, CO, etc. In addition, the materials and circuit layout have been discovered to improve the performance of these particular gas applications. Various metal materials deposited on top of the dielectric substrate, including gold, silver, platinum, etc., could provide desired work potential and interface properties.

Conductive materials are coated upon the electrode of the present disclosure to complete the current circuit for the characterization of electron flow. A conducting polymer, e.g., polyaniline, is the interacting film for the present gas sensor. The attachment of a gas molecule with different affinity alters the electrical properties of the film and is reflected in the current measurement. The electrode layout is also reported to affect the performance of the current characterization. Many alternative layouts prepared for gas analyzer devices are shown in the drawings.

Fabrication of electrodes for gas characterization: A planar micro-supercapacitor configuration can be selected in which interdigitated electrodes are fabricated on the same plane and isolated by physical separation. This configuration has the advantages of easy fabrication and flexibility due to the electrode choice. Figure 18 shows a non-limiting example of a fabrication procedure for the sensors of the present device. The patterned electrodes are fabricated via three primary steps: photoresist deposition, patterning, and pyrolysis. Five mm thick alumina nitrile substrate is used to electrically isolate the supercapacitor from the substrate. SU-8 photoresist (MicroChem) is spun coat to an approximate thickness of 10-100 $\mu$m on the substrate. Following 5 min soft bake at 115 °C in air, the interdigitated electrodes are patterned via UV lithography. The chip is transported to a sputter coating device, after exposure to gold, silver, or platinum plasma PVD treatment under Ar (argon). Current collectors were then deposited using the evaporation of a 100 nm Ti / 400 nm Au layer, annealed at 250°C for 20 min, and patterned with a conventional lift-off process to remove photoresist and blocked metal.

The electrode used in any sensor of the present disclosure can comprise thick film printed interdigitated platinum, gold, or silver lines on a 0.6 mm thick alumina substrate, which provides excellent adhesion with printed wires. Such an electrode is used by spreading material over the interdigitated fingers. The bonding pads allow wires to be contacted for electrical connection.

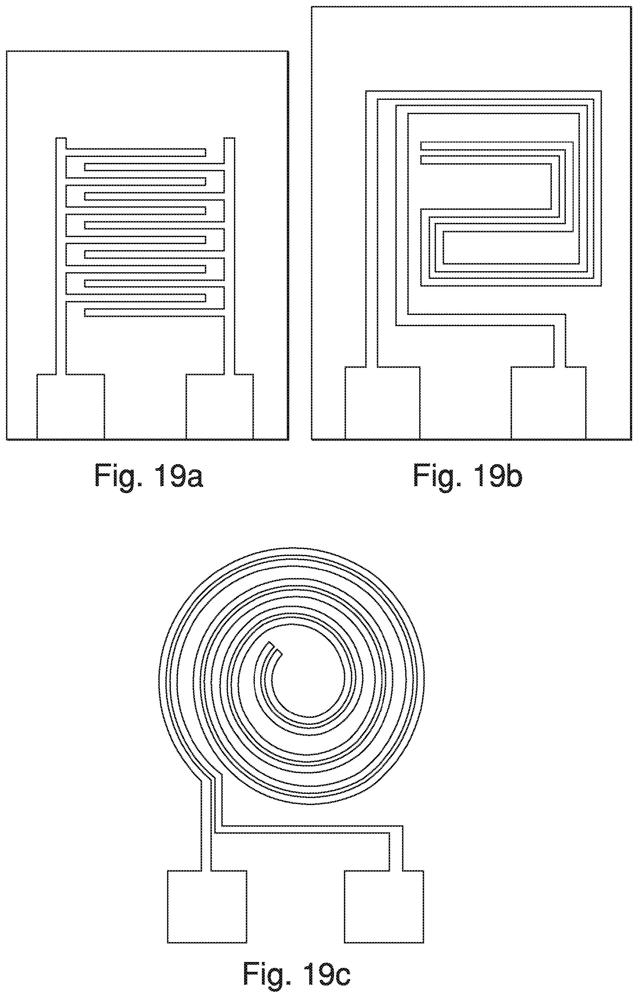

Electrode Layout: The circuit layout determines the performance and consistency of the conductivity measurement. Multiple circuit layouts of interdigitated electrodes are described below and shown in Figs. 19a-19c. Any of these electrode layouts can be used in any (or all) sensors of the present invention.

The electrodes can have a single-channel or multi-channel arrangement. One possible electrode design is a finger electrode as shown in Figure 19a. The gap between the electrodes determines the overall current flow rate with a desired value of 1$\mu$A. Another electrode layout (shown in Fig. 19b) includes two extended wires formed into a rectangle configuration. Yet another electrode layout (shown in Fig. 19c) includes two wires extended into a spiral configuration. Alternative electrode configurations are also contemplated and within the scope of the present disclosure.

In another embodiment of the present invention, the $NH_3$ and $CO_2$ or $^{13}CO_2$ sensors are placed each in its own enclosed chamber within the device. In this embodiment the breath sample is introduced into the device through the mouthpiece and each sensor receives part of the same breath sample. The enclosed chamber for each sensor allows for placement of filters to block specific gases and/or of desiccant directly in the vicinity of each sensor without influencing the response of the other sensors to the same breath content of $NH_3$, $CO_2$ or $^{13}CO_2$.

In some embodiments, the breathalyzer device can connect to and communicate with a remote device such as a smartphone, a wireless hub, a computer, or a server in the cloud using one or multiple communications technologies, including but not limited to, wired communication such as USB, wireless and cellular communication such as WiFi, Bluetooth, ZigBee, GSM, LTE, and infrared. The data may be transmitted as plain text or after being encrypted.

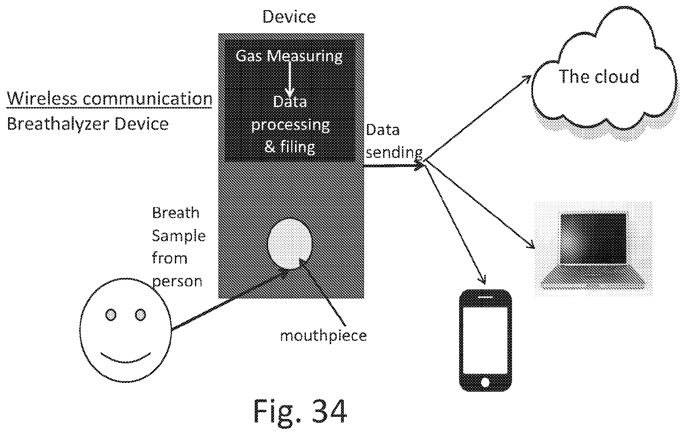

FIG. 34 shows a non-limiting example of a wireless communication breathalyzer device. A breath sample is exhaled into the mouthpiece of the breathalyzer device and is processed by the breathalyzer. The result is then filed and sent to a computer, one or more smart devices, or the cloud.

In some embodiments of the breathalyzer device, the data communicated from the breathalyzer device include data from direct measurement of gas concentrations, as well as device health information such as device temperature and battery status. The data communicated from the breathalyzer device can be raw data read directly from the sensor(s), or can be data processed at the breathalyzer device after signal conditioning. The data communicated can also be derived data such as patient health status, state of a disease patient have, or well-being data.

In some embodiments of the breathalyzer device, the data communicated to the breathalyzer device from a smartphone, a wireless hub, a computer, or a server in the cloud may include device software updates, device parameter updates, and pertinent information about the user or the user's health, well-being or disease status.

## Claims

1. A breath analyzer, comprising:

   an input that receives a breath sample;
   a first sensor that contacts the breath sample, wherein the first sensor comprises a first conductive polymer and a conductive material, wherein the first conductive polymer contacts the conductive material, wherein the first conductive polymer has a resistivity that decreases in response to increased concentration of ammonia;
   a second sensor that contacts the breath sample, wherein the second sensor comprises a second conductive polymer and a conductive material, wherein the second conductive polymer contacts the conductive material of the second sensor, wherein the second conductive polymer has a resistivity that decreases in response to increased concentration of $^{12}CO_2$ and $^{13}CO_2$, wherein the second conductive polymer comprises sulfonated polyaniline blended with polyethylene oxide;
   a processor; and
   an electrical circuit, wherein the electrical circuit operably connects the first and second sensors to the processor, wherein the processor detects resistivity in the electrical circuit and uses the resistivity to calculate a total concentration of ammonia and a total concentration of $^{12}CO_2$ and $^{13}CO_2$ in the breath sample.

2. The breath analyzer of claim 1 wherein the sulfonated polyaniline is synthesized from an emeraldine form of polyaniline polymer.

3. The breath analyzer of claim 1 or claim 2 wherein the conductive material of the first sensor and/or the second sensor comprises a plurality of electrodes.

4. The breath analyzer of claim 3 wherein the plurality of electrodes comprise wires arranged in a spiral configuration.

5. The breath analyzer of claim 3 wherein the plurality of electrodes comprise wires arranged in a rectangular configuration.

6. The breath analyzer of any of claims 1-5 wherein the breath analyzer is in wireless communication with a remote device.

7. The breath analyzer of any of claims 1-5 wherein the breath analyzer has two separate channels, the breath sample being configured to travel from the input, through the separate channels, to each of the first and second sensors.

8. The breath analyzer of any of claims 1-5 wherein a ratio of polyethylene oxide to sulfonated polyaniline is from 10%-30% by weight.

9. The breath analyzer of claim 8 wherein the ratio of polyethylene oxide to sulfonated poly aniline is about 30% by weight.

## Patentansprüche

1. Atemanalysator, umfassend:

   einen Eingang, der eine Atemprobe empfängt;
   einen ersten Sensor, der mit der Atemprobe in Kontakt kommt, wobei der erste Sensor ein erstes leitfähiges Polymer und ein leitfähiges Material umfasst, wobei das erste leitfähige Polymer mit dem leitfähigen Material in Kontakt kommt, wobei das erste leitfähige Polymer einen spezifischen Widerstand aufweist, der als Reaktion auf eine erhöhte Ammoniakkonzentration abnimmt;
   einen zweiten Sensor, der mit der Atemprobe in Kontakt kommt, wobei der zweite Sensor ein zweites leitfähiges Polymer und ein leitfähiges Material umfasst, wobei das zweite leitfähige Polymer mit dem leitfähigen Material des zweiten Sensors in Kontakt kommt, wobei das zweite leitfähige Polymer einen spezifischen Widerstand aufweist, der in Reaktion auf eine erhöhte Konzentration von $^{12}CO_2$ and $^{13}CO_2$ abnimmt, wobei das zweite leitfähige Polymer sulfoniertes Polyanilin in Mischung mit Polyethylenoxid umfasst;

einen Prozessor; und

einen elektrischen Schaltkreis, wobei der elektrische Schaltkreis den ersten und den zweiten Sensor betriebsmäßig mit dem Prozessor verbindet, wobei der Prozessor den spezifischen Widerstand in dem elektrischen Schaltkreis erfasst und anhand des spezifischen Widerstands die Gesamtkonzentration von Ammoniak und die Gesamtkonzentration von $^{12}CO_2$ and $^{13}CO_2$ in der Atemprobe berechnet.

**2.** Atemanalysator nach Anspruch 1, wobei das sulfonierte Polyanilin aus einer Smaragdform des Polyanilinpolymers synthetisiert wird.

**3.** Atemanalysator nach Anspruch 1 oder Anspruch 2, wobei das leitfähige Material des ersten Sensors und/oder des zweiten Sensors eine Mehrzahl von Elektroden umfasst.

**4.** Atemanalysator nach Anspruch 3, wobei die Mehrzahl von Elektroden Drähte umfasst, die in einer spiralförmigen Konfiguration angeordnet sind.

**5.** Atemanalysator nach Anspruch 3, wobei die Mehrzahl von Elektroden Drähte umfasst, die in einer rechteckigen Konfiguration angeordnet sind.

**6.** Atemanalysator nach einem der Ansprüche 1-5, wobei der Atemanalysator in drahtloser Kommunikation mit einem entfernten Gerät steht.

**7.** Atemanalysator nach einem der Ansprüche 1-5, wobei der Atemanalysator über zwei getrennte Kanäle verfügt und die Atemprobe so konfiguriert ist, dass sie vom Eingang durch die getrennten Kanäle zu jedem der ersten und zweiten Sensoren gelangt.

**8.** Atemanalysator nach einem der Ansprüche 1-5, wobei das Verhältnis von Polyethylenoxid zu sulfoniertem Polyanilin zwischen 10 Gew.-%-30 Gew.-% liegt.

**9.** Atemanalysator nach Anspruch 8, wobei das Verhältnis von Polyethylenoxid zu sulfoniertem Polyanilin etwa 30 Gew.-% beträgt.

**Revendications**

**1.** Analyseur d'haleine, comprenant :

une entrée qui reçoit un échantillon d'haleine ;

un premier capteur qui entre en contact avec l'échantillon d'haleine, dans lequel le premier capteur comprend un premier polymère conducteur et un matériau conducteur, dans lequel le premier polymère conducteur entre en contact avec le matériau conducteur, dans lequel le premier polymère conducteur a une résistivité qui diminue en réponse à une concentration accrue d'ammoniac ;

un second capteur qui entre en contact avec l'échantillon d'haleine, dans lequel le second capteur comprend un second polymère conducteur et un matériau conducteur, dans lequel le second polymère conducteur entre en contact avec le matériau conducteur du second capteur, dans lequel le second polymère conducteur a une résistivité qui diminue en réponse à une concentration accrue de $^{12}CO_2$ et $^{13}CO_2$, dans lequel le second polymère conducteur comprend de la polyaniline sulfonée mélangée à de l'oxyde de polyéthylène ;

un processeur ; et

un circuit électrique, dans lequel le circuit électrique relie de manière opérationnelle les premier et second capteurs au processeur, dans lequel le processeur détecte une résistivité dans le circuit électrique et utilise la résistivité pour calculer une concentration totale d'ammoniac et une concentration totale de $^{12}CO_2$ et $^{13}CO_2$ dans l'échantillon d'haleine.

**2.** Analyseur d'haleine selon la revendication 1, dans lequel la polyaniline sulfonée est synthétisée à partir d'une forme émeraude de polymère de polyaniline.

3. Analyseur d'haleine selon la revendication 1 ou la revendication 2 dans lequel le matériau conducteur du premier capteur et/ou du second capteur comprend une pluralité d'électrodes.

4. Analyseur d'haleine selon la revendication 3, dans lequel la pluralité d'électrodes comprennent des fils agencés dans une configuration en spirale.

5. Analyseur d'haleine selon la revendication 3, dans lequel la pluralité d'électrodes comprennent des fils agencés dans une configuration rectangulaire.

6. Analyseur d'haleine selon l'une quelconque des revendications 1 à 5, dans lequel l'analyseur d'haleine est en communication sans fil avec un dispositif distant.

7. Analyseur d'haleine selon l'une quelconque des revendications 1 à 5, dans lequel l'analyseur d'haleine a deux canaux distincts, l'échantillon d'haleine étant conçu pour voyager à partir de l'entrée, à travers les canaux distincts, vers chacun des premiers et seconds capteurs.

8. Analyseur d'haleine selon l'une quelconque des revendications 1 à 5, dans lequel le rapport entre l'oxyde de polyéthylène et la polyaniline sulfonée est de 10 % à 30 % en poids.

9. Analyseur d'haleine selon la revendication 8, dans lequel le rapport entre l'oxyde de polyéthylène et la polyaniline sulfonée est d'environ 30 % en poids.

**Fig. 1** PANI: The acid - base (ES-BS) transition renders it pH sensitive.

Interdigitated platinum
Fingers (IDA)

Contact
pads

**Fig. 2** Interdigitated platinum finger electrodes with spun cast PANI-CSA on top.

Fig. 3 Change in sensor conductivity

Fig. 4 Change in current corresponds with change in conductivity (Fig. 3)

**DropSens flow cell setup**

Top

Center

Sensor

Breath sample injector    Port

Fig. 5 DropSens flow cell

Fig. 6 Calibration plot for $CO_2$
Change in conductivity at 10%
is at 0.2%

Fig. 7 Overall sensitivities of PANI-CSA sensor

Fig. 8 $CO_2$ sensor. PPY-DBSA coated on gold finger electrode.

Fig. 9 PPY-DBSA sensor change in current when exposed to 5% $CO_2$

Fig. 10 PPY-DBSA sensor calibration

Fig. 11 Effect of $CO_2$, $O_2$, $H_2O$ and air on PPY-DBSA sensor
Change in resistivity of PPY-DBSA in the presence of 100% $CO_2$, $O_2$, $H_2O$ and air

Fig. 12 Bench Prototype Schematic

Fig. 13 Illustration of diverting valve for multi-sensor system

DIVERTING

ONE INLET. TWO OUTLETS

Fig. 14 Illustration of diverting valve for multi-sensor system.

Fig. 15 Sensor contact pads connected to Agilent Analyzer through clips and copper wire

Fig. 16 Portable Breathalyzer

Fig. 17 The illustration of filmed figure electrode for the gas sensor.

Fig. 18 The scheme of fabrication of interdigitated electrode.

Fig. 19a

Fig. 19b

Fig. 19c

Pictures of Sensors

**Fig. 20** PPY-DBSA and PANI-CSA on gold electrodes

Comparison of 5% $CO_2$ effect

ASA: PPY with 3-Aminobenzenesulfonic acid

HBSA: PPY with 4-hydroxybenzenesulfonic acid

DBSA: PPY with 4-Dodecylbenzenesulfonic acid

Fig. 21

PPY/ASA             PANI/CSA

3-Aminobenzenesulfonic acid (ASA)

Fig. 22

Electrochemical Polymerization

Anodic Current

←Cathodic Time→←— Anodic Time —→←Cathodic Time→

Cathodic Current

Cathodic Current

Current (A)

| Segment 1 | Segment 2 | Segment 3 |

0

Time (s)

Fig. 23

PPY-ASA: PPY doped with 3-Aminobenzenesulfonic acid

PANI-DNNSA: PANI doped with dinonylnaphthalenesulfonic acid (DNNSA)

Fig. 24

Fig. 25

Fig. 26

Fig. 27

# CO$_2$ characterized by CO$_2$ meter sensor

Fig. 28

Fig. 29

CO$_2$ Bench Prototype

Fig. 30

Fig. 31

PANI/ABSA

PPY/ABSA

Fig. 32

Fig. 33

## Device

Gas Measuring

Data processing & filing

Wireless communication
Breathalyzer Device

Data sending

The cloud

Breath Sample from person

mouthpiece

# Fig. 34

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019065550 W
- US 62879345